# FASCICULE DE BREVET EUROPEEN

(11) **EP 3 898 557 B1**
(45) Date de publication et mention de la délivrance du brevet: **08.02.2023**
(21) Numéro de dépôt: 19817723.0
(22) Date de dépôt: 13.12.2019
(51) Int. Cl.: C07C 1/207, B01J 38/02, B01J 38/14, B01J 35/00, B01J 23/92, B01J 23/72, B01J 23/20, B01J 21/08, C07C 47/06, C07C 45/00, C07C 11/167, B01J 38/24

(54) **PROCÉDÉ DE PRODUCTION DE BUTADIÈNE À PARTIR D'ÉTHANOL AVEC RÉGÉNÉRATION IN SITU OPTIMISÉE DU CATALYSEUR DE LA DEUXIÈME ÉTAPE RÉACTIONNELLE**
VERFAHREN ZUR HERSTELLUNG VON BUTADIEN AUS ETHANOL MIT OPTIMIERTER IN-SITU-REGENERATION DES KATALYSATORS DES ZWEITEN REAKTIONSSCHRITTES
METHOD FOR PRODUCING BUTADIENE FROM ETHANOL WITH OPTIMISED IN SITU REGENERATION OF THE CATALYST OF THE SECOND REACTION STEP

(30) Priorité: 21.12.2018 FR 1873771
(43) Date de publication de la demande: 27.10.2021
(73) Titulaire: IFP Energies nouvelles, 92852 Rueil-Malmaison (FR); COMPAGNIE GENERALE DES ETABLISSEMENTS MICHELIN, 63000 Clermont-Ferrand (FR)
(72) Inventeur: DROBYSHEV, Kirill, 92852 RUEIL-MALMAISON (FR); DASTILLUNG, Rejane, 92852 RUEIL-MALMAISON (FR); GABELLE, Jean-Christophe, 92852 RUEIL-MALMAISON (FR); ROLLAND, Matthieu, 92852 RUEIL-MALMAISON (FR)
(74) Mandataire: IFP Energies nouvelles
(86) Numéro de dépôt international: PCT/EP2019/085113
(87) Numéro de publication internationale: WO 2020/126920

(56) Documents cités:
- WO-A2-2017/091771
- FR-A1- 3 026 100
- US-A- 2 357 365

## Description

### Domaine technique

L'invention concerne un procédé de production de butadiène à partir d'éthanol opérant en deux étapes réactionnelles, une première étape de conversion de l'éthanol en acétaldéhyde et une seconde étape de conversion d'un mélange d'éthanol et d'acétaldéhyde en butadiène, cette seconde étape étant réalisée dans plusieurs réacteurs fonctionnant en parallèle, en présence d'un catalyseur, régénéré in situ.

### Technique antérieure

Les procédés de production de butadiène à partir d'éthanol ont été développés, en particulier, par les Russes sur la base des travaux de Lebedev dans les années 20 (procédé en 1 étape réactionnelle), et par les Américains durant la seconde guerre mondiale à partir des travaux d'Ostromilenski (procédé en 2 étapes réactionnelles : déshydrogénation d'éthanol en acétaldéhyde, puis production du butadiène à partir d'un mélange éthanol/acétaldéhyde). Ce procédé en deux étapes permet d'obtenir des rendements un peu meilleurs. Il a été opéré pendant les années 40 aux États-Unis mais toutes les unités de ce type ont été arrêtées pour des raisons principalement économiques.

Les procédés de production de butadiène à partir d'éthanol, dans la version Lebedev (1 étape) ou Ostromilenski (2 étapes), ont une conversion par passe inférieure à 50%.

Un autre problème du procédé est la production d'une grande variété d'impuretés de toutes sortes : des hydrocarbures saturés, insaturés, et aromatiques, mais aussi des produits oxygénés tels que des alcools, des phénols, des aldéhydes, des cétones, des acides, des esters, des éthers, des acétals.

En effet, derrière la réaction globale de transformation de l'éthanol en butadiène se cachent de nombreuses réactions chimiques comprenant une réaction de déshydrogénation permettant de générer de l'acétaldéhyde (I), une réaction d'aldolisation/crotonisation de l'acétaldéhyde en crotonaldéhyde (II), une réaction de Merwein-Pondorff-Verley (MPV) entre l'éthanol et le crotonaldéhyde (III) et finalement une étape de déshydratation de l'alcool crotylique en butadiène (IV).

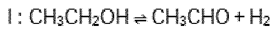

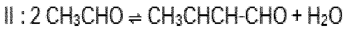

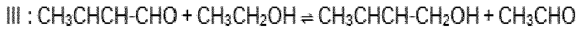

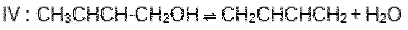

Cette multiplicité de réactions chimiques est à l'origine de nombreux sous-produits si l'enchainement des étapes ne se fait pas dans l'ordre précisé ci-dessus, avec notamment la présence de réactions de déshydratation et de condensation secondaires. De plus, d'autres réactions peuvent se produire (comme l'isomérisation, la cyclisation, la réaction de Diels et Aider, etc.) augmentant encore le nombre de sous-produits.

Même si beaucoup des sous-produits sont entrainés avec l'effluent réactionnel vers des unités de séparation/traitement en aval des réacteurs, comme dans le procédé CARBIDE de production de 1,3-butadiène en deux étapes, en particulier présenté dans le livre « Synthetic rubber », chapitre 4 (W.J. Toussaint et J. Lee Marah), ou plus récemment dans les procédés des brevets FR 3 026 100 et FR 3 026 101, le catalyseur en particulier utilisé lors de la deuxième étape pour la conversion du mélange éthanol-acétaldéhyde en 1,3-butadiène voit son activité baisser rapidement, notamment en quelques semaines, voire quelques jours. Beaucoup de travaux récents se sont attachés à améliorer le rendement en butadiène global du procédé, comme par exemple les travaux décrits dans le brevet français FR 3 026 100, ou à améliorer l'activité et la sélectivité des catalyseurs de la deuxième étape réactionnelle (cf. par exemple FR 3 038 851).

Plus particulièrement, le brevet FR 3 026 100 décrit un procédé de production en deux étapes de butadiène à partir d'une charge éthanol issue de sources renouvelables, qui permet d'éliminer les impuretés tout en minimisant la perte en éthanol et acétaldéhyde et en maximisant ainsi le rendement global du procédé. Le procédé décrit permet également de limiter la consommation des utilités réduisant de fait la consommation énergétique du procédé. Cependant, le brevet FR 3 026 100 est silencieux quant à un système de régénération notamment du catalyseur de la deuxième étape réactionnelle.

Il existe peu, voire pas, de travaux relatifs à la régénération des catalyseurs de conversion du mélange éthanol/acétaldéhyde en butadiène. Il apparait donc indispensable de développer une méthode de régénération des catalyseurs en particulier de la deuxième étape réactionnelle de conversion de l'éthanol en butadiène, tout en maximisant le rendement en butadiène.

Le brevet FR 1 294 619 décrit un procédé de régénération simultanée d'un catalyseur d'un système de déshydrogénation cyclique à réacteurs multiples à lit fixe, le catalyseur étant régénéré sous flux d'air et en présence d'un gaz combustible tel que le gaz naturel (ou un gaz équivalent composé d'hydrocarbures C₁-C₄), sans recyclage du gaz de régénération.

Le brevet US 2,357,365 décrit un procédé de conversion-régénération dans un système multi-réacteurs, les réacteurs fonctionnant par paire, l'un étant en phase opérationnelle pendant que le deuxième est en régénération. La régénération du catalyseur est opérée de façon simultanée dans au moins deux réacteurs, sous un flux gazeux composé d'azote et d'oxygène à une teneur de 1-2% mol., ledit flux gazeux circulant dans une boucle de régénération.

Le brevet US 2,915,570 décrit la régénération d'un catalyseur de conversion d'hydrocarbures en butadiène, sous flux d'air à au moins 540°C, ledit catalyseur étant à base de chrome sur un support alumine.

Une des contraintes du procédé de conversion de l'éthanol en butadiène en deux étapes réactionnelles est la complexité du protocole de régénération en particulier des catalyseurs de la deuxième étape. En effet, le protocole de régénération, bien connu de l'Homme du métier, comprend un enchainement de phases de balayage sous flux de gaz inerte à différentes teneurs en oxygène. Cet enchainement de phases de balayage de flux gazeux de compositions différentes devient un problème d'autant plus difficile à résoudre que la durée du cycle catalytique des catalyseurs, en particulier à base de tantale, utilisés pour la deuxième étape, est courte.

La présente invention vise à résoudre ces problèmes de régénération sans impacter la productivité en butadiène. Plus particulièrement, la présente invention vise à proposer un procédé de production du butadiène, en deux étapes réactionnelles, comprenant une méthode optimisée de régénération in situ du catalyseur de la deuxième étape réactionnelle, ledit procédé permettant d'assurer la production en continu de butadiène avec un rendement maximisé et de diminuer fortement les fluctuations de la composition en sortie des réacteurs et donc du débit en butadiène produit.

### Résumé de l'invention

L'invention concerne un procédé de production de butadiène à partir d'éthanol, comprenant au moins les étapes suivantes :
a) une étape de conversion de l'éthanol en acétaldéhyde, pour produire un effluent éthanol/acétaldéhyde, comprenant au moins une section réactionnelle (A) alimentée par un flux comprenant de l'éthanol et opérée en présence d'un catalyseur (Ca) ;
b) une étape de conversion en butadiène comprenant au moins une section réactionnelle-régénérative dans laquelle sont mises en oeuvre simultanément une étape réactionnelle et une étape de régénération, dans (n+n/2) réacteurs à lit fixe, n étant un nombre entier égal à 4 ou à l'un de ses multiples, lesdits (n+n/2) réacteurs à lit fixe comprenant chacun au moins un lit fixe d'un catalyseur (Cb), lesdits (n+n/2) réacteurs à lit fixe fonctionnant en parallèle et en séquencé de telle sorte que ladite étape réactionnelle débute dans chacun desdits réacteurs avec un décalage dans le temps égal au quart de la durée de cycle catalytique dudit catalyseur (Cb), ladite section réactionnelle-régénérative comprenant trois boucles de régénération, et de façon à ce que, à chaque instant :
   b1) ladite étape réactionnelle est opérée dans n desdits réacteurs à lit fixe, n étant un nombre entier égal à 4 ou à l'un de ses multiples, alimentés au moins par une fraction dudit effluent éthanol/acétaldéhyde issu de l'étape a), à une température comprise entre 300 et 400°C, à une pression comprise entre 0,1 et 1,0 MPa, et pendant un temps égal à la durée du cycle catalytique dudit catalyseur (Cb), pour produire un effluent réactionnel, et
   b2) ladite étape de régénération est opérée, dans n/2 desdits réacteurs à lit fixe, pendant une durée totale égale à la moitié de la durée de cycle catalytique dudit catalyseur (Cb), et comprend les quatre phases successives suivantes :
      i. une phase de stripage opérée à une température comprise entre 300 et 400°C, sous un flux de gaz inerte, ladite phase i) débutant à l'issue de l'étape réactionnelle b1); puis
      ii. une première phase de combustion opérée à l'issue de la phase i) sous un flux gazeux comprenant ledit gaz inerte et de l'oxygène à une teneur inférieure ou égale à 1%vol. par rapport au volume total dudit flux gazeux, à une température comprise entre 300 et 450°C ; puis
      iii. une deuxième phase de combustion opérée à l'issue de la première phase de combustion ii) sous un flux gazeux comprenant ledit gaz inerte et de l'oxygène à une teneur supérieure ou égale à 2%vol. par rapport au volume total dudit flux gazeux, à une température comprise entre 390 et 550°C ; puis
      iv. une phase de stripage final opérée à une température comprise entre 550°C et 300°C, sous un flux dudit gaz inerte;
lesdites trois boucles de régénération de ladite section réactionnelle-régénérative comprenant une boucle de régénération du gaz inerte, une boucle de régénération du flux gazeux de la première phase de combustion ii) et une boucle de régénération du flux gazeux de la deuxième phase de combustion iii).

Le procédé selon l'invention, qui utilise la voie de conversion en deux étapes de l'éthanol en butadiène, permet la régénération in situ du catalyseur de la deuxième étape de conversion. Un des intérêts de cette régénération in situ réside dans le fait qu'elle permet de compenser au moins en partie la problématique d'encrassage rapide du catalyseur utilisé lors de la deuxième étape. La régénération du procédé selon l'invention suit des conditions opératoires relativement douces pour éliminer le coke déposé sur le catalyseur, évitant ainsi la dégradation prématurée du catalyseur tout en permettant de très bonnes performances de régénération, c'est-à-dire tout en permettant une combustion performante du coke déposé sur le catalyseur de la deuxième étape réactionnelle.

Un des avantages du procédé selon l'invention réside dans le fait qu'il permet d'assurer la production en continu de butadiène et, ceci, avec des fluctuations de la composition de l'effluent produit faibles et donc des variations réduites du débit en butadiène produit. En effet, dans la mesure où il utilise un système multi-réacteurs en particulier pour la deuxième étape de conversion, avec des réacteurs fonctionnant en parallèle, et permet le passage rapide dans un réacteur du mode opérationnel au mode de régénération, et inversement du mode de régénération au mode opérationnel, le procédé selon l'invention permet d'assurer un débit constant de l'effluent en sortie des unités réactionnelles et de lisser la composition de l'effluent produit tout au long de la production. Dans les conditions du procédé selon l'invention, la production de butadiène est assurée en continu.

Bien qu'il comprenne un système de réaction-régénération en continu, le procédé selon l'invention qui compte trois boucles de régénération, permet également une forte économie en utilités. Il permet notamment de limiter la consommation en gaz inerte, tel que l'azote, qui est recyclé au moins en partie. Le procédé selon l'invention permet aussi une optimisation économique du procédé de production du butadiène à partir de l'éthanol en deux étapes réactionnelles.

Le procédé de la présente invention peut, très avantageusement, être intégré dans un procédé de conversion de l'éthanol en butadiène, complexe comprenant une ou plusieurs unités de séparation et/ou de purification, permettant de produire un butadiène très pur et de récupérer les réactifs non convertis ou convertis partiellement et de les recycler vers les réacteurs. Un tel procédé est par exemple très bien décrit dans le brevet français FR 3 026 100.

### Description des modes de réalisation

Selon la présente invention, par « système multi-réacteurs » ou « système de réacteurs multiples », on entend un ensemble de (n+n/2) réacteurs à lit fixe, n étant un nombre entier égal à 4 ou à l'un de ses multiples, soit un ensemble d'au moins 6 (soit 4+2) réacteurs, fonctionnant en parallèle et de façon séquencée, c'est-à-dire telle que l'étape réactionnelle, notamment l'étape réactionnelle b1), débute dans chacun des réacteurs dudit système multi-réacteurs avec un décalage dans le temps les uns par rapport aux autres. De préférence, le système multi-réacteurs de l'étape b) du procédé de l'invention, comprend donc 6 réacteurs à lit fixe, c'est-à-dire que l'entier n est égal à 4. En pratique, dans le cas où le système multi-réacteurs de l'étape b) du procédé de l'invention comprend 6 réacteurs (R1, R2, R3, R4, R5 et R6), l'étape réactionnelle b1) débute d'abord dans le réacteur R1, puis dans le réacteur R2 après un certain temps, noté t, puis dans R3 après le même temps t par rapport à R2, et ainsi de suite jusqu'au réacteur R6, les temps de décalage étant tous égaux au temps t de décalage entre R1 et R2. Les étapes réactionnelle b1) et régénérative b2) s'enchainant successivement et de façon cyclique dans chaque réacteur, la régénération débute également de façon séquencé dans chacun des réacteurs. Cependant, à un instant donné, comme les réacteurs fonctionnent en parallèle, les étapes de réaction et de régénération sont simultanées dans le système multi-réacteurs à (n+n/2) réacteurs.

Selon la présente invention, les réacteurs à lit fixe sont dits « non opérationnels » ou « en mode non opérationnel », dès que l'étape de régénération b2) débute et jusqu'à la fin de ladite étape de régénération, en particulier jusqu'à la fin de la phase iv) de stripage final. Inversement, les réacteurs à lit fixe sont dits « opérationnels » ou « en mode opérationnel », lorsque les réactions de conversion du mélange éthanol/acétaldéhyde en butadiène sont mises en oeuvre dans lesdits réacteurs à lit fixe, c'est-à-dire dès que l'étape réactionnelle b1) débute jusqu'à la fin du cycle catalytique du catalyseur (Cb) dans lesdits réacteurs à lit fixe. De manière très avantageuse, pendant l'étape b) de conversion en butadiène du procédé selon l'invention, il y a simultanément n réacteurs à lit fixe en mode opérationnel et n/2 réacteurs à lit fixe en mode non opérationnel.

Selon la présente invention, l'expression « régénération en boucle » signifie que les gaz de régénération de l'étape b2) du procédé de l'invention sont traités, purifiés et recyclés au moins en partie. Ainsi l'étape de régénération b2) met en oeuvre trois boucles de régénération des gaz de régénération. Avantageusement, l'étape de régénération b2) comprend une boucle de régénération du gaz inerte, une boucle de régénération du flux gazeux à faible teneur en oxygène et une boucle de régénération du flux gazeux à forte teneur en oxygène. Chaque boucle de régénération comprend de préférence un système de traitement et purification du flux gazeux correspondant. Chaque boucle de régénération peut indépendamment comprendre une purge permettant d'éliminer d'éventuels déchets résultant de la combustion de coke.

Selon l'invention, on entend par « cycle catalytique » d'un catalyseur, en particulier du catalyseur (Cb) de la deuxième étape réactionnelle, la phase opérationnelle dudit catalyseur au cours de laquelle ce dernier joue pleinement son rôle dans la réaction de conversion notamment du mélange éthanol/acétaldéhyde en butadiène. De préférence, le cycle catalytique du catalyseur (Cb) correspond à la phase opérationnelle dudit catalyseur lorsqu'un minimum de 16% mol., de préférence de 23%.mol., de conversion de l'éthanol par passe sur ledit catalyseur (Cb) de la deuxième étape réactionnelle (c'est-à-dire l'étape réactionnelle b1) du procédé selon l'invention) est assuré.

Selon la présente invention, lorsque la température est dite « constante », il est bien entendu que c'est la température de consigne qui est constante, la température réelle pouvant oscillée autour de cette valeur de consigne.

De même, une rampe de montée ou de descente en température signifie que la température de consigne subit une montée ou une descente en température, les températures réelles oscillant autour de la température de consigne.

Selon la présente invention, l'expression « compris entre ... et ... » signifie que les valeurs limites de l'intervalle sont incluses dans la gamme de valeurs décrite. Si tel n'était pas le cas et que les valeurs limites n'étaient pas incluses dans la gamme décrite, une telle précision sera apportée par la présente invention.

Ainsi, l'invention concerne un procédé de production de butadiène à partir d'éthanol à deux étapes réactionnelles, la deuxième étape de conversion d'un mélange éthanol/acétaldéhyde en butadiène comprenant un enchainement d'étapes de réaction et de régénération dans un système de réacteurs multiples, les réacteurs fonctionnant en parallèle et en séquencé.

Plus particulièrement, l'invention concerne un procédé de production de butadiène à partir d'éthanol, comprenant au moins les étapes suivantes :
a) une étape de conversion de l'éthanol en acétaldéhyde, pour produire un effluent éthanol/acétaldéhyde, comprenant au moins une section réactionnelle (A) alimentée par un flux comprenant de l'éthanol et opérée en présence d'un catalyseur (Ca), de préférence ladite étape de conversion de l'éthanol en acétaldéhyde est mise en oeuvre à une température comprise entre 200 et 500°C, préférentiellement entre 250°C et 300°C, et à une pression comprise entre 0,1 et 1,0 MPa, préférentiellement entre 0,1 et 0,5 MPa, de manière plus préférée entre 0,1 et 0,3 MPa;
b) une étape de conversion en butadiène comprenant au moins une section réactionnelle-régénérative dans laquelle sont mises en oeuvre simultanément une étape réactionnelle et une étape de régénération, dans (n+n/2) réacteurs à lit fixe, n étant un nombre entier égal à 4 ou à l'un de ses multiples, lesdits (n+n/2) réacteurs à lit fixe comprenant chacun au moins un lit fixe d'un catalyseur (Cb), de préférence à base de tantale, lesdits (n+n/2) réacteurs à lit fixe fonctionnant en parallèle et en séquencé de telle sorte que ladite étape réactionnelle débute dans chacun desdits réacteurs avec un décalage dans le temps égal au quart de la durée de cycle catalytique dudit catalyseur (Cb), ladite section réactionnelle-régénérative comprenant trois boucles de régénération, et de façon à ce que, à chaque instant :
   b1) ladite étape réactionnelle est opérée dans n desdits réacteurs à lit fixe, n étant un nombre entier égal à 4 ou à l'un de ses multiples, alimentés au moins par une fraction dudit effluent éthanol/acétaldéhyde issu de l'étape a), à une température comprise entre 300 et 400°C, de préférence entre 300 et 360°C, à une pression comprise entre 0,1 et 1,0 MPa, de préférence entre 0,2 et 0,4 MPa, et pendant un temps égal à la durée du cycle catalytique dudit catalyseur (b), pour produire un effluent réactionnel, et
   b2) ladite étape de régénération est opérée, dans n/2 desdits réacteurs à lit fixe, avantageusement non opérationnels, pendant une durée totale égale à la moitié de la durée de cycle catalytique dudit catalyseur (Cb), et comprend, de préférence consiste en, les quatre phases successives suivantes :
      i. une phase de stripage opérée à une température comprise entre 300 et 400°C, de préférence entre 330 et 370°C, sous un flux de gaz inerte, de préférence d'azote, de dioxyde de carbone (CO₂) ou leurs mélanges, et de manière très préférée sous un flux d'azote, avantageusement à un débit compris entre 0,5 et 1,5 Nm³/h/kg de catalyseur, de préférence égal à 1 Nm³/h/kg de catalyseur, ladite phase i) débutant à l'issue de l'étape réactionnelle b1); puis
      ii. une première phase de combustion opérée à l'issue de la phase i), sous flux gazeux comprenant ledit gaz inerte et de l'oxygène (en particulier sous forme de dioxygène O₂) à une teneur inférieure ou égale à 1%vol. par rapport au volume total dudit flux gazeux, de préférence à une teneur comprise entre 0,1 et 1%vol., préférentiellement entre 0,3 et 0,7%vol. par rapport au volume total dudit flux gazeux, à une température comprise entre 300 et 450°C, de préférence entre 330 et 430°C, en particulier à une température constante comprise entre 330 et 370°C suivie d'une rampe de montée en température de 10 à 30°C/h puis d'une phase à température constante comprise entre 390 et 430°C, et avantageusement à un débit en ledit flux gazeux compris entre 1,7 et 2,5 Nm³/h/kg de catalyseur, de préférence entre 1,9 et 2,3 Nm³/h/kg de catalyseur; puis
      iii. une deuxième phase de combustion opérée à l'issue de la première phase de combustion ii), sous flux gazeux comprenant ledit gaz inerte et de l'oxygène (en particulier sous forme de dioxygène O₂) à une teneur supérieure ou égale à 2%vol. par rapport au volume total dudit flux gazeux, de préférence à une teneur comprise entre 2 et 20%vol., préférentiellement entre 2 et 10%vol., plus préférentiellement entre 4 et 8%vol. par rapport au volume total dudit flux gazeux, à une température comprise entre 390 et 550°C, de préférence à une température constante comprise entre 390 et 430°C suivie d'une rampe de montée en température de 10 à 30°C/h puis d'une phase à température constante comprise entre 460 et 510°C, et avantageusement à un débit en ledit flux gazeux compris entre 1,2 et 1,8 Nm³/h/kg de catalyseur, de préférence entre 1,35 à 1,50 Nm3/h/kg de catalyseur; puis
      iv. une phase de stripage final opérée à une température comprise entre 550 et 300°C, de préférence selon une rampe de descente en température de 50 à 150°C/h suivie d'une phase à température constante comprise entre 300 et 400°C, de préférence entre 330 et 370°C, sous un flux dudit gaz inerte, de préférence d'azote, de dioxyde de carbone (CO₂) ou leurs mélanges, et de manière très préférée sous un flux d'azote, avantageusement à un débit compris entre 0,5 et 1,5 Nm³/h/kg de catalyseur, de préférence égal à 1 Nm³/h/kg de catalyseur;
lesdites trois boucles de régénération de ladite section réactionnelle-régénérative comprenant une boucle de régénération du gaz inerte, une boucle de régénération du flux gazeux de la première phase de combustion ii) et une boucle de régénération du flux gazeux de la deuxième phase de combustion iii).

De préférence, le procédé selon l'invention comprend la succession, en particulier dans cet ordre, de l'étape a) de conversion de l'éthanol en acétaldéhyde et de l'étape b) de conversion en butadiène, sans étape intermédiaire.

Très avantageusement, le procédé selon l'invention peut être intégré dans un procédé plus global de conversion de l'éthanol en butadiène, comprenant en particulier des étapes de séparation et de purification en aval des réacteurs de conversion, par exemple tel que le procédé décrit dans le brevet français FR 3 026 100.

### La charge

Conformément à l'invention, le procédé convertit l'éthanol en butadiène.

Avantageusement, le flux comprenant l'éthanol qui alimente le procédé selon l'invention comprend au moins 80% poids d'éthanol, préférentiellement au moins 90% poids, et de manière préférée au moins 93 % poids. De manière très préférée, la charge éthanol comprenant ledit flux répond aux spécifications d'éthanol carburant EN 15376. Ladite charge éthanol peut provenir de toute origine, fossile, végétale ou animale, et en particulier des procédés de production d'éthanol à partir de ressources végétale.

### Etape a) de conversion de l'éthanol en acétaldéhyde

Conformément à l'invention, le procédé comprend une étape a) de conversion de l'éthanol en acétaldéhyde, pour produire un effluent éthanol/acétaldéhyde avantageusement sous forme liquide. Ladite étape a) du procédé selon l'invention comprend au moins une section réactionnelle (A) alimentée par un flux comprenant de l'éthanol.

Ladite étape a) de conversion de l'éthanol en acétaldéhyde est opérée en présence d'un catalyseur (Ca), avantageusement d'un catalyseur de déshydrogénation des alcools. Tout catalyseur, notamment tout catalyseur de déshydrogénation des alcools, en particulier de l'éthanol, connu de l'Homme du métier peut être utilisé dans l'étape a) du procédé selon l'invention. De manière très préférée, le catalyseur (Ca) de ladite étape a) du procédé selon l'invention est un catalyseur de déshydrogénation de l'éthanol comprenant un élément déshydrogénant, plus particulièrement un élément cuivre, plus préférentiellement un oxyde de cuivre ou un mélange d'oxyde de chrome et d'oxyde de cuivre, et une matrice en particulier à base de silice.

Avantageusement, l'étape a) du procédé de l'invention est opérée dans des conditions de température et de pression connues de l'Homme du métier. De préférence, la section réactionnelle (A) de l'étape a) du procédé de l'invention est opérée à une température comprise entre 200 et 500°C, préférentiellement entre 250°C et 300°C, et à une pression comprise entre 0,1 et 1,0 MPa, préférentiellement entre 0,1 et 0,5 MPa, de manière plus préférée entre 0,1 et 0,3 MPa.

Ladite étape a) du procédé selon l'invention permet avantageusement de convertir l'éthanol en acétaldéhyde par déshydrogénation de l'éthanol. De préférence, la conversion de l'éthanol en acétaldéhyde est comprise entre 25 et 40% par passe, avec une sélectivité comprise entre 85 et 100% vers l'acétaldéhyde, de manière préférée comprise entre 90 et 97% vers l'acétaldéhyde.

En plus de l'acétaldéhyde, la réaction de déshydrogénation qui est mise en oeuvre lors de l'étape a) du procédé selon l'invention produit de l'hydrogène. Ainsi, de manière préférée, l'étape a) du procédé de l'invention comprend en outre une section de séparation pour séparer au moins un effluent hydrogène sous forme gazeuse et ledit effluent éthanol/acétaldéhyde sous forme liquide. Lorsqu'elle est intégrée à ladite étape a) du procédé selon l'invention, ladite section de séparation éventuelle met en oeuvre des moyens de séparation gaz-liquide connus de l'Homme du métier. De préférence, ladite section de séparation comprend un séparateur gaz-liquide opéré à une pression comprise entre 0,1 et 0,3 MPa, et une température comprise entre 25 et 60°C.

### Etape b) de conversion en butadiène

Conformément à l'invention, l'étape b) de conversion en butadiène du procédé de l'invention comprend au moins une section réactionnelle-régénérative dans laquelle une étape réactionnelle et une étape de régénération sont mises en oeuvre simultanément. Ladite section réactionnelle-régénérative comprend un système de réacteurs multiples comprenant (n+n/2) réacteurs à lit fixe, n étant un nombre entier égal à 4 ou à l'un de ses multiples, de préférence n étant un nombre entier égal à 4, lesdits (n+n/2) réacteurs à lit fixe fonctionnant en parallèle et en séquencé. Ladite section réactionnelle-régénérative comprend également trois boucles de régénération et de préférence un système de conduits et de vannes qui permet la circulation des flux gazeux de régénération dans chacun des (n+n/2) réacteurs de ladite section réactionnelle-régénérative. Plus particulièrement, ladite section réactionnelle-régénérative comprend une boucle de régénération de gaz inerte et deux boucles de régénération de flux gazeux comprenant de l'oxygène.

Si à un instant donné, lesdites étapes réactionnelle b1) et régénérative b2) sont mises en oeuvre simultanément dans ledit système de réacteurs multiples, c'est-à-dire dans l'ensemble des (n+n/2) réacteurs à lit fixe de ladite section réactionnelle-régénérative, elles sont mises en oeuvre successivement et de façon cyclique dans chacun desdits réacteurs à lit fixe.

Conformément à l'invention, l'étape b) est une étape de conversion en butadiène d'un mélange éthanol et acétaldéhyde, ledit mélange comprenant au moins une fraction dudit effluent éthanol/acétaldéhyde issu de l'étape a).

Selon l'invention, chaque réacteur à lit fixe comprend au moins un lit fixe d'un catalyseur (Cb). Ledit catalyseur (Cb) comprend avantageusement un élément sélectionné dans le groupe constitué du tantale, zirconium et de colombium (ou niobium), de préférence sous leur forme oxyde. De préférence, le catalyseur (Cb) comprend l'élément tantale, et de manière préférée un oxyde de tantale. Avantageusement, le catalyseur (Cb) comprend également un support, de préférence une matrice mésoporeuse, de manière préférée à base de silice. Par exemple, ledit catalyseur (Cb) est le catalyseur décrit dans la demande FR 3 038 851. La durée de cycle catalytique dudit catalyseur (Cb) est, en particulier, supérieure ou égale à 1 jour, de préférence supérieure ou égale à 6 jours, et inférieure ou égale à 20 jours, plus particulièrement inférieure ou égale à 15 jours. Par exemple, la durée de cycle du catalyseur (Cb) du procédé selon l'invention est égale à 10 jours. Cette durée permet d'assurer une conversion de l'éthanol par passe sur ledit catalyseur (Cb), au moins égale à 16%mol., de préférence au moins égale à 23%mol.

Le fonctionnement en séquencé desdits (n+n/2) réacteurs, c'est-à-dire le décalage dans le temps de fonctionnement desdits réacteurs, est ajusté tel que l'étape réactionnelle b1) débute dans chacun desdits réacteurs avec un décalage dans le temps égal au quart de la durée de cycle catalytique dudit catalyseur (Cb), de sorte que l'étape réactionnelle b1) débute le réacteur Rᵢ₊₁ avec un décalage dans le temps égal au quart de la durée de cycle catalytique dudit catalyseur (Cb) par rapport au réacteur Rᵢ, avec i un nombre entier variant de 1 à ((n+n/2)-1). Par exemple, dans le cas où le système multi-réacteurs comprend 6 réacteurs (4+2 réacteurs) et où la durée de cycle catalytique est de 10 jours, l'étape réactionnelle b1) débute d'abord dans le réacteur R1 puis dans le réacteur R2 2,5 jours après avoir débuté dans le réacteur R1, puis dans le réacteur R3 2,5 jours après avoir débuté dans le réacteur R2, puis dans le réacteur R4 2,5 jours après avoir débuté dans le réacteur R3, et ainsi de suite jusqu'au réacteur R6.

Conformément à l'invention, l'étape b) comprend une étape réactionnelle b1) pour produire un effluent réactionnel, comprenant avantageusement du butadiène.

Avantageusement, lesdits réacteurs à lit fixe mis en oeuvre dans ladite étape réactionnelle b1) selon l'invention, c'est-à-dire lesdites réacteurs opérationnels, sont alimentée par au moins une fraction dudit effluent éthanol/acétaldéhyde issu de l'étape a). Eventuellement, lesdits réacteurs à lit fixe opérationnels mis en oeuvre dans ladite étape réactionnelle b1) selon l'invention peuvent en outre être alimentée par un appoint en éthanol, comprenant notamment un flux éthanol recyclé, et/ou un appoint en acétaldéhyde, comprenant notamment un flux acétaldéhyde recyclé, lesdits flux éthanol et acétaldéhyde recyclés étant issus d'unités de purification-séparation éventuellement présentes en aval du procédé selon l'invention, et dans lesquels les réactifs (éthanol acétaldéhyde) non convertis sont avantageusement séparés. Lorsque ladite étape réactionnelle b1) selon l'invention comprend un appoint additionnel en éthanol et/ou en acétaldéhyde, les débits d'alimentation de ladite étape réactionnelle b1), en particulier en ladite au moins une fraction dudit effluent éthanol/acétaldéhyde issu de l'étape a) et en les éventuels appoints en éthanol et en acétaldéhyde, sont avantageusement réglés de telle sorte que le rapport molaire entre la quantité molaire totale d'éthanol par rapport à la quantité totale molaire d'acétaldéhyde en entrée des réacteurs à lit fixe opérationnels de ladite étape réactionnelle b1)est compris entre 1 et 5, de manière préférée entre 2 et 4 et de manière très préférée entre 2,4 et 3,6. Selon l'invention, ladite étape réactionnelle b1) est mise en oeuvre dans n desdits réacteurs à lit fixe, n étant un nombre entier égal à 4 ou à l'un de ses multiples, fonctionnant en parallèle et en séquencé. Avantageusement, ladite étape réactionnelle b1) est opérée à une température comprise entre 300 et 400°C, de préférence entre 300 et 360°C, à une pression comprise entre 0,1 et 1,0 MPa, de préférence entre 0,2 et 0,4 MPa, et pendant un temps égal à la durée du cycle catalytique dudit catalyseur (Cb). De préférence, ladite étape réactionnelle b1) est opérée à une vitesse pondérale horaire (PPH) comprise entre 0,8 et 2,5 h⁻¹, de préférence entre 0,8 et 2 h⁻¹, la vitesse pondérale horaire (PPH, poids par poids horaire) étant définie comme le rapport entre le débit massique de charge éthanol/acétaldéhyde entrant dans les n réacteurs opérationnels et la masse de catalyseur compris dans lesdits n réacteurs opérationnels.

Avantageusement, ladite étape réactionnelle b1) permet de convertir au moins une partie du mélange éthanol/acétaldéhyde en butadiène. La sélectivité en butadiène de cette étape réactionnelle est de préférence supérieure à 60%, de manière préférée supérieure à 70%, de manière très préférée supérieure à 80%. Par sélectivité en butadiène, on entend le rapport molaire du débit de butadiène dans l'effluent réactionnel issu de ladite étape réactionnelle b1) sur le débit du mélange d'éthanol et d'acétaldéhyde consommé dans ladite étape réactionnelle b1), exprimé en pourcentage. La conversion du mélange éthanol/acétaldéhyde est de préférence supérieure à 20%, de manière préférée supérieure à 25%, de manière préférée supérieure à 30%. Par conversion du mélange éthanol/acétaldéhyde, on entend le rapport molaire entre le débit molaire d'éthanol et d'acétaldéhyde consommé dans ladite étape réactionnelle b1), c'est-à-dire l'écart de débit molaire du mélange éthanol/acétaldéhyde entre l'alimentation et l'effluent réactionnel issu de ladite étape réactionnelle b1), et le débit molaire du mélange éthanol/acétaldéhyde dans l'alimentation de ladite étape réactionnelle b1), exprimé en pourcentage. De préférence, au moins 35%, de préférence entre 35 et 80%, de manière très préférée entre 40 et 80% de l'acétaldéhyde est converti dans ladite section réactionnelle.

Eventuellement, l'étape b) du procédé selon l'invention peut comprendre en outre une section de séparation pour séparer l'effluent réactionnel issu de l'étape réactionnelle b1) en au moins un effluent gazeux et un effluent liquide. Ladite section de séparation met en oeuvre des moyens de séparation gaz-liquide connus de l'Homme du métier. De manière préférée, est utilisé un séparateur gaz-liquide opéré à une pression comprise entre 0,1 et 0,3 MPa, et une température comprise entre 25 et 60°C. De préférence, ledit effluent gazeux est traité ultérieurement audit procédé selon l'invention pour produire un flux de butadiène purifié et l'effluent liquide est traité pour produire notamment un flux riche en éthanol non converti et/ou un flux riche en acétaldéhyde. Ledit flux riche en éthanol non converti peut être avantageusement recyclé en amont de l'étape a) de conversion de l'éthanol en acétaldéhyde et/ou en amont de l'étape b) de conversion en butadiène, à l'issue de l'étape a). Ledit flux riche en acétaldéhyde peut être avantageusement recyclé en amont de l'étape b) et à l'issue de l'étape a) du procédé de l'invention.

Conformément à l'invention, ladite section réactionnelle-régénérative de l'étape b) de conversion en butadiène comprend une étape b2) de régénération, en particulier de régénération en boucle, dans les réacteurs à lit fixe non opérationnels. A un instant donné, n/2 desdits réacteurs à lit fixe de ladite section réactionnelle-régénérative sont non opérationnel. Dans chaque réacteur de ladite section réactionnelle-régénérative, ladite étape b2) est opérée à l'issue de l'étape réactionnelle b1), pendant une durée égale à la moitié de la durée de cycle catalytique dudit catalyseur (Cb). Par exemple, lorsque la durée de cycle catalytique du catalyseur (Cb) est de 10 jours, la durée de l'étape b2) de régénération est de 5 jours.

Ladite étape b2) de régénération est une régénération in situ du catalyseur (Cb) utilisé lors de la deuxième étape réactionnelle de transformation de l'éthanol en butadiène, c'est-à-dire utilisé lors de l'étape réactionnelle b1) du procédé selon l'invention. Il est connu que cette deuxième étape réactionnelle (étape de conversion d'un mélange éthanol/acétaldéhyde en butadiène) implique une multiplicité de réactions chimiques qui peut être à l'origine de nombreux sous-produits et donc d'encrassement rapide du catalyseur. Ainsi, le cycle catalytique du catalyseur (Cb), en particulier à base de tantale, de cette deuxième étape réactionnelle est généralement assez court, notamment inférieur ou égal à 20 jours, plus particulièrement inférieur ou égal à 15 jours, par exemple égal à 10 jours, et le taux de coke déposé sur ledit catalyseur en fin de cycle catalytique est relativement élevé. Ainsi une régénération in situ, de préférence en boucle, du catalyseur (Cb) dans les réacteurs multiples de l'étape b) du procédé selon l'invention apparait indispensable pour assurer la production de butadiène en continu, tout en limitant la consommation d'utilités, en particulier du gaz inerte, tel que l'azote, nécessaire en quantités importantes notamment pour limiter l'exothermie du brûlage du coke, lors de l'étape de régénération.

Conformément à l'invention, l'étape de régénération b2) du catalyseur (Cb) comprend, de préférence consiste en, quatre phases successives opérées dans les n/2 réacteurs à lit fixe non opérationnels.

Il y a, d'abord, une phase de stripage i) qui débute, dans un réacteur à lit fixe, à l'issue de l'étape réactionnelle b1). Ladite étape de stripage i) est opérée à une température comprise entre 300 et 400°C, de préférence entre 330 et 370°C, de manière très préférée entre 340°C et 360°C, sous un flux de gaz inerte. Le gaz inerte est l'azote, le dioxyde de carbone (CO₂) ou leurs mélanges, et de préférence l'azote. Avantageusement, le débit de gaz inerte est compris entre 0,5 et 1,5 Nm³/h/kg de catalyseur et, de préférence, est égal à 1 Nm³/h/kg de catalyseur. De manière très préférée, cette phase i) de stripage dure entre 2 et 20%, de préférence entre 5 et 15%, de la durée totale de ladite étape b2) de régénération, soit entre 1 et 10%, de préférence entre 3 et 7%, de la durée du cycle catalytique du catalyseur (Cb) de l'étape b) du procédé selon l'invention.

Cette phase i) de stripage initial est suivie d'une première phase de combustion ii), dite en conditions O₂ faible (ou en termes anglais Low O₂), au cours de laquelle le coke mou est calciné. Par coke mou, on entend les composés du coke déposé sur le catalyseur dont la température de dégradation est relativement faible, en particulier inférieure à 400°C, comme par exemples les composés résultant de l'oligomérisation des oléfines notamment en C4. Pendant la première phase de combustion ii), il peut également y avoir un début de calcination du coke dur, qui regroupe des composés hydrocarbonés dont la température de dégradation est plus élevée, en particulier supérieure ou égale à 400°C et inférieure ou égale à 450°C. Ladite première phase de combustion ii) est opérée sous un flux gazeux comprenant ledit gaz inerte et de l'oxygène (sous forme d'O₂) à une teneur inférieure ou égale à 1%vol. par rapport au volume total dudit flux gazeux, de préférence à une teneur comprise entre 0,1 et 1%vol., préférentiellement entre 0,3 et 0,7%vol. par rapport au volume total dudit flux gazeux. Ladite première phase de combustion ii) est avantageusement opérée à une température comprise entre 300 et 450°C, de préférence entre 330 et 430°C, en particulier à une température constante comprise entre 330 et 370°C suivie d'une rampe de montée en température de 10 à 30°C/h puis d'une phase à température constante comprise entre 390 et 430°C. Avantageusement, le débit dudit flux gazeux, comprenant le gaz inerte et de l'oxygène (sous forme d'O₂) à une teneur inférieure ou égale à 1%vol., est compris entre 1,7 et 2,5 Nm³/h/kg de catalyseur, de préférence entre 0,9 et 2,3 Nm³/h/kg de catalyseur. De manière très préférée, ladite première phase de combustion ii) dure entre 5 et 40%, de préférence entre 10 et 30%, de la durée totale de ladite étape b2) de régénération, soit entre 2 et 20%, de préférence entre 5 et 15%, de la durée du cycle catalytique du catalyseur (Cb) de l'étape b) du procédé selon l'invention.

Une deuxième phase de combustion iii), dite en conditions O₂ élevé (ou en termes anglais High O₂), est effectuée à l'issue de la première phase de combustion ii) pour brûler le coke résiduel, c'est-à-dire les composés du coke dont les températures de dégradation sont les plus élevées, notamment supérieures à 450°C. Ladite deuxième phase de combustion iii) est opérée sous flux gazeux comprenant ledit gaz inerte et de l'oxygène (sous forme d'O₂) à une teneur supérieure ou égale à 2%vol. par rapport au volume total dudit flux gazeux, de préférence à une teneur comprise entre 2 et 20%vol., préférentiellement entre 2 et 10%vol., plus préférentiellement entre 4 et 8%vol. par rapport au volume total dudit flux gazeux. Ladite deuxième phase de combustion iii) est avantageusement opérée à une température comprise entre 390 et 550°C, de préférence à une température constante comprise entre 390 et 430°C suivie selon une rampe de montée en température de 10 à 30°C/h puis d'une phase à température constante comprise entre 460 et 510°C. Avantageusement, le débit dudit flux gazeux, comprenant le gaz inerte et de l'oxygène (sous forme d'O₂) à une teneur supérieure ou égale à 2%vol., est compris entre 1,2 et 1,8 Nm³/h/kg de catalyseur, de préférence compris entre 1,35 à 1,50 Nm3/h/kg de catalyseur. De manière très préférée, ladite deuxième phase de combustion iii) dure entre 5 et 40%, de préférence entre 10 et 30%, de la durée totale de ladite étape b2) de régénération, soit entre 2 et 20%, de préférence entre 5 et 15%, de la durée du cycle catalytique du catalyseur (Cb) de l'étape b) du procédé selon l'invention.

Enfin, l'étape b2) de régénération comprend une phase iv) de stripage final, effectuée à l'issue de la deuxième phase de combustion iii), dans chacun desdits n/2 réacteurs à lits fixes, en particulier non opérationnels, sous un flux dudit gaz inerte, de préférence d'azote, de dioxyde de carbone (CO₂) ou leurs mélanges, et de manière très préférée sous un flux d'azote, avantageusement à un débit compris entre 0,5 et 1,5 Nm³/h/kg de catalyseur, de préférence égal à 1 Nm³/h/kg de catalyseur. Ladite phase iv) de stripage final est avantageusement opérée à une température comprise entre 550 et 300°C, de préférence selon une rampe de descente en température de 50 à 150°C/h suivie d'une phase à température constante comprise entre 300 et 400°C, de préférence entre 330 et 370°C. De manière très préférée, ladite phase de stripage final iv) dure entre 2 et 20%, de préférence entre 5 et 15%, de la durée totale de ladite étape b2) de régénération, soit entre 1 et 10%, de préférence entre 3 et 7%, de la durée du cycle catalytique du catalyseur (Cb) de l'étape b) du procédé selon l'invention. Avantageusement, les phases i) et iv) de stripage permettent d'éliminer les produits de combustion résiduels des réacteurs avant et après la combustion du coke.

Les desdites phases i), ii), iii) et iv) de ladite étape de régénération b2) sont opérées successivement et dans cet ordre dans chacun desdits (n+n/2) réacteurs à lits fixes, et avantageusement à l'issue de l'étape réactionnelle b1).

A l'issue de la phase de stripage final iv) de l'étape de régénération b2), les réacteurs à lit fixe qui ont subi l'étape b2) de régénération sont considérés opérationnels et l'étape réactionnelle b1) peut avantageusement être mise en oeuvre dans ces réacteurs, dits régénérés. Ainsi, dans chacun desdits (n+n/2) réacteurs à lit fixe de la section réactionnelle-régénérative du procédé selon l'invention, l'étape réactionnelle b1) et l'étape de régénération b2) sont mises en oeuvre successivement et avantageusement de façon cyclique. Parallèlement, à un instant donné du procédé de l'invention, l'étape réactionnelle b1) et l'étape de régénération b2) sont opérées simultanément dans le système multi-réacteurs mis en oeuvre dans le procédé selon l'invention.

Très avantageusement, ladite section réactionnelle-régénérative de l'étape b) du procédé selon l'invention comprend trois boucles de régénération des flux gazeux. De préférence, ladite section réactionnelle-régénérative de l'étape b) du procédé selon l'invention comprend également un système de conduits et de vannes permettant de relier lesdits (n+n/2) réacteurs à lits fixe aux boucles de régénération, d'éviter la circulation des flux gazeux de régénération dans les réacteurs opérationnels et de faire circuler les flux gazeux adaptés aux phases de régénération de l'étape b2) dans les réacteurs non opérationnels.

En particulier, ladite section réactionnelle-régénérative de l'étape b) du procédé selon l'invention comprend trois boucles de régénération des flux gazeux. Chacun desdits flux gazeux de régénération peut ainsi indépendamment être récupéré en sortie des réacteurs à lits fixe non opérationnels, purgé et/ou purifié pour éliminer les composés résultant de la combustion du coke, comme par exemple le CO, CO₂, l'eau, séparé et recyclé au moins en partie. Ainsi, en fonction de la phase de régénération, au moins en partie un flux de gaz inerte, de manière très préférée un flux d'azote, un flux gazeux à faible teneur en oxygène (c'est-à-dire à une teneur en O₂ inférieure ou égale à 1%vol. par rapport au volume total dudit flux gazeux) ou un flux gazeux à forte teneur en oxygène (c'est-à-dire à une teneur en O₂ supérieure ou égale à 2%vol. par rapport au volume total dudit flux gazeux) peut être récupéré, traité et recyclé. Chaque boucle de régénération comprend, de préférence, une purge et/ou un appoint en flux gazeux correspondant.

De préférence, ladite section réactionnelle-régénérative de l'étape b) comprend avantageusement une boucle de régénération de gaz inerte pour récupérer, traiter et recycler au moins en partie les flux gazeux en sortie de réacteur pendant les phases i) et iv) de stripage, et deux boucles de régénération de flux gazeux comprenant de l'oxygène, pour récupérer, traiter et recycler au moins en partie les flux gazeux en sortie de réacteur pendant les phases ii) et iii) de combustion, l'une permettant de traiter et recycler au moins en partie le flux gazeux à faible teneur en O₂ (inférieure ou égale à 1%vol. par rapport au volume total dudit flux gazeux) nécessaire pour la première phase ii) de combustion et l'autre permettant de traiter et recycler au moins en partie le flux gazeux à forte teneur en O₂ (supérieure ou égale à 2%vol. par rapport au volume total dudit flux gazeux) nécessaire pour la deuxième phase iii) de combustion.

Ainsi, le procédé selon l'invention permet d'assurer la production de butadiène en continu, avec des variations de composition de l'effluent réactionnel limitées et donc des fluctuations de production de butadiène faibles (c'est-à-dire des fluctuations faibles du débit de butadiène produit), tout en limitant la consommation d'utilités, en particulier la consommation de gaz inerte tel que l'azote. Le procédé selon l'invention peut également permettre un gain économique d'un procédé de production de butadiène à partir de l'éthanol, à deux étapes réactionnelles, en particulier d'un procédé plus global du type de celui décrit par exemple dans le brevet FR 3 026 100.

Les Figures intégrées à la présente description et les exemples qui suivent sont présentés à titre illustratif et non limitatif du procédé selon l'invention.

### Liste des figures

[Fig 1]

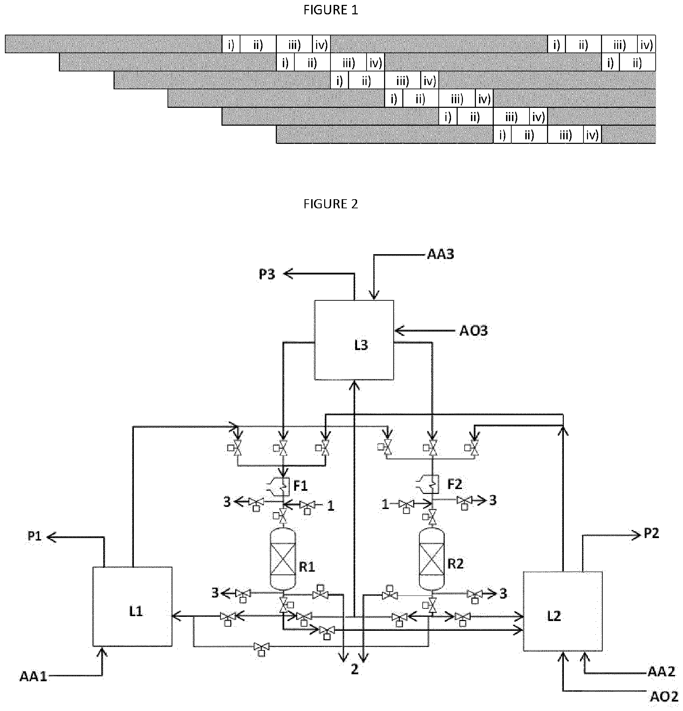

   La Figure 1 représente de manière schématique et non limitative un organigramme de fonctionnement des réacteurs à lit fixe dans un système à 6 réacteurs à lit fixe, lors de l'étape b) de conversion en butadiène, une ligne correspondant eu fonctionnement d'un réacteur, les étapes en gris foncé correspondant aux étapes réactionnelles b1), les étapes en gris clair correspondant aux différentes phases de l'étape b2) de régénération : les phases i) correspondant aux phases de stripage initial i), les phases ii) correspondant aux premières phases de combustion ii), les phases iii) correspondant aux deuxièmes phases de combustion iii) et les phases iv) correspondant aux phases de stripage final iv).
[Fig 2]
   La Figure 2 représente le schéma de l'étape de régénération b2) dans 2 réacteurs (R1) et (R2) en mode non opérationnel (c'est-à-dire dans un système multi-réacteurs comprenant 6 réacteurs à lit fixe), comprenant trois boucles régénératives, une boucle (L1) de régénération du gaz inerte, une boucle (L2) de régénération du flux gazeux à faible teneur en oxygène et une boucle (L3) de régénération du flux gazeux à forte teneur en oxygène. Chaque boucle (L1), (L2) et (L3) de régénération comprend une purge, respectivement (P1), (P2) et (P3). La boucle (L1) de régénération du gaz inerte (ou boucle de stripage) comprend également un appoint (AA1) en gaz inerte. La boucle (L2) de régénération du flux gazeux à faible teneur en oxygène (ou boucle Low O₂) comprend un appoint (AA2) en gaz inerte et un appoint en dioxygène (AO2) (par exemple sous forme d'apport en air) et la boucle (L3) de régénération du flux gazeux à forte teneur en oxygène (ou boucle High O₂) comprend aussi un appoint (AA3) en gaz inerte et un appoint en dioxygène (AO3) (par exemple sous forme d'apport en air). Avant les réacteurs (R1) et (R2), en phase de régénération, les flux gazeux peuvent être réchauffés dans des fours (F1) et (F2). Les conduits (3) correspondent à des piquages de sécurité. Les conduits (1) représentent les conduits d'alimentation en charge éthanol/acétaldéhyde des réacteurs (R1) et (R2) lorsque ceux-ci sont opérationnels. Les conduits (2) représentent les conduits en sortie de réacteurs (R1) et (R2) par lesquels l'effluent réactionnel produit est récupéré pour alimenter par exemple une section de séparation.

### Exemples

Les exemples suivants sont basés sur des simulations de procédés intégrant des données thermodynamiques calées sur des points expérimentaux.

Dans chacun des exemples suivants, le procédé décrit est intégré dans un procédé plus global comme celui décrit dans le brevet français FR 3 026 100. La charge éthanol du procédé global est issue de source renouvelable et comprend plus de 93% poids d'éthanol. Le débit de charge alimentant le procédé global est ajustée de manière à obtenir une production annuelle de 150 kt/an d'un butadiène ayant une pureté comprise entre 99,5 et 100% poids (en adéquation avec l'utilisation actuelle du produit), avec une durée de fonctionnement annuelle du procédé de 8000 h.

Dans les exemples suivants, on entend par « variation(s) de composition », la(ou les) amplitudes moyennes de variation des teneurs pondérales des composés de l'effluent réactionnel, sur la durée de fonctionnement.

### Exemple 1 (conforme)

Dans cet exemple, la deuxième unité réactionnelle comprend 6 (4+2) réacteurs à lit fixe et est opérée à une vitesse pondérale horaire (PPH) de 2 h⁻¹. La section de régénération comprend 3 boucles de régénération.

La conversion de l'éthanol en acétaldéhyde est effectuée dans un réacteur multitubulaire comprenant un catalyseur à base d'oxyde de cuivre sur un support silice, à 275°C et 0,26 MPa. L'effluent éthanol/acétaldéhyde, séparé du flux d'hydrogène en sortie de réacteur, est alors envoyé vers la deuxième unité réactionnelle.

La conversion en butadiène est mise en oeuvre dans 6 réacteurs à lit fixe radial, contenant chacun un lit fixe d'un catalyseur à base d'oxyde de tantale sur une matrice à base de silice, à 380°C et 0,2 MPa et à une vitesse pondérale horaire (PPH) de 2 h⁻¹. Des appoints en éthanol et en acétaldéhyde, issus de sections de purification et séparation des flux d'éthanol et d'acétaldéhyde non convertis en aval des sections réactionnelles, sont ajoutés à l'effluent éthanol / acétaldéhyde en entrée de deuxième unité réactionnelle, de telle façon que le débit total en mélange éthanol/acétaldéhyde, à l'entrée de la deuxième unité réactionnelle, est égale à 92,4 t/h. Dans ces conditions, la conversion de l'éthanol est de 34% mol dans l'étape 2 de réaction et le cycle catalytique du catalyseur à base d'oxyde de tantale est de 10 jours. Les 6 réacteurs fonctionnent en parallèle et de manière séquencée comme schématisé sur la Figure 1 : chacun débute la phase réactionnelle avec un écart de 5 jours ; à un instant donné, 4 réacteurs sont opérationnels et 2 réacteurs sont en phase de régénération. Le catalyseur est régénéré in situ dans chaque réacteur selon le protocole présenté dans le Tableau 1. La section de régénération comprend 3 boucles de régénération :
- une boucle à l'azote permettant d'assurer le flux d'azote nécessaire au stripage du catalyseur,
- une boucle de flux gazeux à faible teneur en O₂ permettant d'assurer un flux gazeux comprenant de l'azote et 0,5% vol. d'O₂ et
- une boucle de flux gazeux à faible teneur en O₂ permettant d'assurer un flux gazeux comprenant de l'azote et 6,0% vol. d'O₂.

**Tableau 1 : Protocole de régénération**

| Période | T initiale, °C | T finale, °C | Rampe, °C/h | % vol. O₂ | Durée de palier, h |
|---|---|---|---|---|---|
| I | 350 | 350 | - | 0,0 | 12 |
| II | 350 | 350 | - | 0,5 | 30 |
| III | 350 | 410 | 20 | 0,5 | 3 |
| IV | 410 | 410 | - | 0,5 | 16,5 |
| V | 410 | 410 | - | 6,0 | 19 |
| VI | 410 | 480 | 20 | 6,0 | 3,5 |
| VII | 480 | 480 | - | 6,0 | 20 |
| VIII | 480 | 350 | 100 | 0,0 | 1,5 |
| IX | 350 | 350 | - | 0,0 | 12 |

Le Tableau 2 indique des variations de compositions dans l'effluent de la réaction, sur la durée de fonctionnement.

**Tableau 2 : Variations de compositions dans l'effluent de la réaction**

| Composant | Variation, % pds |
|---|---|
| Ethanol | 0,8 |
| Acétaldéhyde | 4,7 |
| Butadiène | 6,1 |
| Diethylether | 6,7 |

Il apparait que les variations de composition de l'effluent réactionnel observées en sortie d'unité réactionnelle sont faibles. Sur la durée de fonctionnement du procédé, la production en butadiène présente des variations de débit de 6% poids seulement.

Le Tableau 3 présente les consommations en utilités pour la régénération du catalyseur à base d'oxyde de tantale, de l'ensemble des réacteurs, sur la durée de fonctionnement.

**Tableau 3 : Consommations des utilités pour la régénération**

| | |
|---|---|
| Azote, Nm³/h | 48,79 |
| Air Instrument, Nm3/h | 317,19 |
| Electricité, kW | 2084,25 |
| Eau de chaudière, t/h | 2,67 |
| Fuel gaz, kW | 1923,95 |

La consommation en utilités pour l'étape de régénération est relativement faible. En particulier, la consommation en azote (48,79 Nm³/h) sur la durée de fonctionnement du procédé reste très faible notamment par rapport à la consommation en azote d'un système de réacteurs similaire mais avec une régénération once-through (N₂ consommé = 42 400 Nm³/h).

### Exemple 2 (conforme)

Dans cet exemple, la deuxième unité réactionnelle comprend 6 (4+2) réacteurs à lit fixe et est opérée à une vitesse pondérale horaire (PPH) de 1,2 h⁻¹ . La section de régénération comprend 3 boucles de régénération.

La conversion de l'éthanol en acétaldéhyde est effectuée dans un réacteur multitubulaire comprenant un catalyseur à base d'oxyde de cuivre sur un support silice, à 275°C et 0,26 MPa. L'effluent éthanol/acétaldéhyde, séparé du flux d'hydrogène en sortie de réacteur, est alors envoyé vers la deuxième unité réactionnelle.

La conversion en butadiène est mise en oeuvre dans 6 réacteurs à lit fixe radial, contenant chacun un lit fixe d'un catalyseur à base d'oxyde de tantale sur une matrice à base de silice, à 380°C et 0,2 MPa et à une vitesse pondérale horaire (PPH) de 1,2 h⁻¹. Des appoints en éthanol et en acétaldéhyde, issus de sections de purification et séparation des flux d'éthanol et d'acétaldéhyde non convertis en aval des sections réactionnelles, sont ajoutés à l'effluent éthanol / acétaldéhyde en entrée de deuxième unité réactionnelle, de telle façon que le débit total en mélange éthanol/acétaldéhyde, à l'entrée de la deuxième unité réactionnelle, est égale à 129,7 t/h. Dans ces conditions, la conversion de l'éthanol est de 23% mol dans l'étape 2 de réaction et le cycle catalytique du catalyseur à base d'oxyde de tantale est de 10 jours. Les 6 réacteurs fonctionnent en parallèle et de manière séquencée comme schématisé sur la Figure 1 : chacun débute la phase réactionnelle avec un écart de 5 jours ; à un instant donné, 4 réacteurs sont opérationnels et 2 réacteurs sont en phase de régénération. Le catalyseur est régénéré in situ dans chaque réacteur selon le même protocole que celui de l'Exemple 1 et présenté dans le Tableau 1. La section de régénération comprend 3 boucles de régénération :
- une boucle à l'azote permettant d'assurer le flux d'azote nécessaire au stripage du catalyseur,
- une boucle de flux gazeux à faible teneur en O₂ permettant d'assurer un flux gazeux comprenant de l'azote et 0,5% vol. et
- une boucle de flux gazeux à faible teneur en O₂ permettant d'assurer un flux gazeux comprenant de l'azote et 6,0% vol. d'O₂

Le Tableau 4 indique des variations de composition dans l'effluent de la réaction.

**Tableau 4 : Variations de compositions dans l'effluent de la réaction**

| Composant | Variation, % pds |
|---|---|
| Ethanol | 0,8 |
| Acétaldéhyde | 4,7 |
| Butadiène | 6,1 |
| Diethylether | 6,7 |

Il apparait que les variations de composition de l'effluent réactionnel observées en sortie d'unité réactionnelle sont faibles, tout au long du fonctionnement. Sur la durée de fonctionnement du procédé, la production en butadiène présente des variations de débit de 6% poids seulement.

Le Tableau 5 présente les consommations en utilités pour la régénération du catalyseur à base d'oxyde de tantale, de l'ensemble des réacteurs, sur la durée de fonctionnement.

**Tableau 5 : Consommations des utilités pour la régénération**

| | |
|---|---|
| Azote, Nm3/h | 59,69 |
| Air Instrument, Nm3/h | 787,89 |
| Electricité, kW | 4658,61 |
| Eau de chaudière, t/h | 3,80 |
| Fuel gaz, kW | 4229,62 |

La consommation en utilités pour l'étape de régénération est relativement faible. En particulier la consommation en azote (59,69 Nm³/h) sur la durée de fonctionnement du procédé reste très faible notamment par rapport à la consommation en azote d'un système de réacteurs similaire mais avec une régénération once-through (N₂ consommé = 47 619 Nm³/h).

### Exemple 3 (non conforme)

Dans cet exemple, le système multi-réacteurs comprend 2 réacteurs : un réacteur est en mode opérationnel pendant que le deuxième est en mode régénération (non opérationnel). Tous les autres paramètres de réaction et de régénération sont similaires à ceux de l'Exemple 1.

Le Tableau 6 indique des variations de compositions dans l'effluent de la réaction.

**Tableau 6 : Variations de compositions dans l'effluent de la réaction**

| Composant | Variation, % pds |
|---|---|
| Ethanol | 4,6 |
| Acétaldéhyde | 22,6 |
| Butadiène | 29,3 |
| Diethylether | 27,4 |

Il apparait que les variations de composition de l'effluent réactionnel sont plus importants en sortie de l'unité à 2 réacteurs comme celui de l'exemple 3 (non conforme) que celles observées dans les systèmes à 6 réacteurs décrits dans les exemples 1 et 2 (conformes). La production de butadiène, dans le procédé décrit dans l'exemple 3, présente des variations de 30% poids.

## Revendications

1. Procédé de production de butadiène à partir d'éthanol, comprenant au moins les étapes suivantes :
a) une étape de conversion de l'éthanol en acétaldéhyde, pour produire un effluent éthanol/acétaldéhyde, comprenant au moins une section réactionnelle (A) alimentée par un flux comprenant de l'éthanol et opérée en présence d'un catalyseur (Ca) ;
b) une étape de conversion en butadiène comprenant au moins une section réactionnelle-régénérative dans laquelle sont mises en oeuvre simultanément une étape réactionnelle et une étape de régénération, dans (n+n/2) réacteurs à lit fixe, n étant un nombre entier égal à 4 ou à l'un de ses multiples, lesdits (n+n/2) réacteurs à lit fixe comprenant chacun au moins un lit fixe d'un catalyseur (Cb), lesdits (n+n/2) réacteurs à lit fixe fonctionnant en parallèle et en séquencé de telle sorte que ladite étape réactionnelle débute dans chacun desdits réacteurs avec un décalage dans le temps égal au quart de la durée de cycle catalytique dudit catalyseur (Cb), ladite section réactionnelle-régénérative comprenant trois boucles de régénération, et de façon à ce que, à chaque instant :
b1) ladite étape réactionnelle est opérée dans n desdits réacteurs à lit fixe, n étant un nombre entier égal à 4 ou à l'un de ses multiples, alimentés au moins par une fraction dudit effluent éthanol/acétaldéhyde issu de l'étape a), à une température comprise entre 300 et 400°C, à une pression comprise entre 0,1 et 1,0 MPa, et pendant un temps égal à la durée du cycle catalytique dudit catalyseur (b), pour produire un effluent réactionnel, et
b2) ladite étape de régénération est opérée, dans n/2 desdits réacteurs à lit fixe, pendant une durée totale égale à la moitié de la durée de cycle catalytique dudit catalyseur (Cb), et comprend les quatre phases successives suivantes :
i. une phase de stripage opérée à une température comprise entre 300 et 400°C, sous un flux de gaz inerte, ladite phase i) débutant à l'issue de l'étape réactionnelle b1); puis
ii. une première phase de combustion opérée à l'issue de la phase i) sous un flux gazeux comprenant ledit gaz inerte et de l'oxygène à une teneur inférieure ou égale à 1%vol. par rapport au volume total dudit flux gazeux, à une température comprise entre 300 et 450°C ; puis
iii. une deuxième phase de combustion opérée à l'issue de la première phase de combustion ii) sous un flux gazeux comprenant ledit gaz inerte et de l'oxygène à une teneur supérieure ou égale à 2%vol. par rapport au volume total dudit flux gazeux, à une température comprise entre 390 et 550°C ; puis
iv. une phase de stripage final opérée à une température comprise entre 550°C et 300°C, sous un flux dudit gaz inerte;
lesdites trois boucles de régénération de ladite section réactionnelle-régénérative comprenant une boucle de régénération du gaz inerte, une boucle de régénération du flux gazeux de la première phase de combustion ii) et une boucle de régénération du flux gazeux de la deuxième phase de combustion iii).

2. Procédé selon la revendication 1, dans lequel la section réactionnelle de l'étape a) est opérée à une température comprise entre 200 et 500°C, préférentiellement entre 250°C et 300°C, et à une pression comprise entre 0,1 et 1,0 MPa, préférentiellement entre 0,1 et 0,5 MPa, de manière plus préférée entre 0,1 et 0,3 MPa.

3. Procédé selon l'une des revendications 1 à 2, dans lequel lesdits réacteurs à lit fixe mis en oeuvre dans ladite étape réactionnelle b1) sont en outre alimentée par un appoint en éthanol et/ou un appoint en acétaldéhyde, les débits d'alimentation étant tels que le rapport molaire entre la quantité molaire totale d'éthanol par rapport à la quantité totale molaire d'acétaldéhyde en entrée desdits réacteurs à lit fixe de ladite étape réactionnelle b1) est compris entre 1 et 5.

4. Procédé selon l'une des revendications 1 à 3, dans lequel le nombre entier n est égal à 4 et ladite section réactionnelle-régénérative de l'étape b) comprend 6 réacteurs à lit fixe.

5. Procédé selon l'une des revendications 1 à 4, dans lequel ladite étape réactionnelle b1) est opérée à une température comprise 300 et 360°C.

6. Procédé selon l'une des revendications 1 à 5, dans lequel ladite étape réactionnelle b1) est opérée à une pression comprise entre 0,2 et 0,4 MPa.

7. Procédé selon l'une des revendications 1 à 6, dans lequel la durée de cycle catalytique dudit catalyseur (Cb) de l'étape b) de conversion en butadiène est supérieure ou égale à 1 jour, de préférence supérieure ou égale à 6 jours, et inférieure ou égale à 20 jours, plus particulièrement préférence inférieure ou égale à 15 jours.

8. Procédé selon l'une des revendications 1 à 7, dans lequel le gaz inerte de l'étape de régénération b2) est l'azote, le dioxyde de carbone (CO₂) ou leurs mélanges, de manière très préférée l'azote.

9. Procédé selon l'une des revendications 1 à 8, dans lequel ladite phase de stripage i) est opérée à une température comprise entre 330 et 370°C.

10. Procédé selon l'une des revendications 1 à 9, dans lequel le débit de gaz inerte de ladite phase de stripage i) est compris entre 0,5 et 1,5 Nm³/h/kg de catalyseur, de préférence égal à 1 Nm³/h/kg de catalyseur.

11. Procédé selon l'une des revendications 1 à 10, dans lequel ladite première phase de combustion ii) est opérée sous flux gazeux comprenant une teneur en oxygène comprise entre 0,1 et 1%vol., de préférence entre 0,3 et 0,7%vol., par rapport au volume total dudit flux gazeux.

12. Procédé selon l'une des revendications 1 à 11, dans lequel ladite première phase de combustion ii) est opérée à une température comprise entre 330 et 430°C, en particulier à une température constante comprise entre 330 et 370°C suivie d'une rampe de montée en température de 10 à 30°C/h puis d'une phase à température constante comprise entre 390 et 430°C.

13. Procédé selon l'une des revendications 1 à 12, dans lequel ladite première phase de combustion ii) est opérée à un débit en flux gazeux compris entre 1,7 et 2,5 Nm³/h/kg de catalyseur, de préférence entre 1,9 et 2,3 Nm³/h/kg de catalyseur.

14. Procédé selon l'une des revendications 1 à 13, dans lequel ladite deuxième phase de combustion iii) est opérée sous flux gazeux comprenant une teneur en oxygène comprise entre 2 et 20%vol., de préférence entre 2 et 10%vol., préférentiellement entre 4 et 8%vol. par rapport au volume total dudit flux gazeux.

15. Procédé selon l'une des revendications 1 à 14, dans lequel ladite deuxième phase de combustion iii) est opérée à une température constante comprise entre 390 et 430°C suivie d'une rampe de montée en température de 10 à 30°C/h puis d'une phase à température constante comprise entre 460 et 510°C.

16. Procédé selon l'une des revendications 1 à 15, dans lequel ladite deuxième phase de combustion iii) est opérée à un débit en flux gazeux compris entre 1,2 et 1,8 Nm³/h/kg de catalyseur, de préférence entre 1,35 à 1,50 Nm3/h/kg de catalyseur.

17. Procédé selon l'une des revendications 1 à 16, dans lequel ladite phase de stripage final iv) est opérée selon une rampe de descente en température de 50 à 150°C/h suivie d'une phase à température constante comprise entre 300 et 400°C, de préférence entre 330 et 370°C.

18. Procédé selon l'une des revendications 1 à 17, dans lequel ladite phase de stripage final iv) est opérée sous un flux dudit gaz inerte, à un débit compris entre 0,5 et 1,5 Nm³/h/kg de catalyseur, de préférence égal à 1 Nm³/h/kg de catalyseur.

## Patentansprüche

1. Verfahren zur Herstellung von Butadien aus Ethanol, das mindestens die folgenden Schritte umfasst:
a) einen Schritt zur Umwandlung von Ethanol zu Acetaldehyd zum Herstellen eines Ethanol/Acetaldehyd-Austrags, der mindestens einen Reaktionsabschnitt (A) umfasst, in den ein Strom geleitet wird, der Ethanol enthält, und in Gegenwart eines Katalysators (Ca) betrieben wird;
b) einen Schritt zur Umwandlung in Butadien, der mindestens einen Reaktions-/Regenerationsabschnitt umfasst, in dem gleichzeitig ein Reaktionsschritt und ein Regenerationsschritt in (n+n/2) Festbettreaktoren ausgeführt werden, wobei n eine Ganzzahl ist, die 4 oder einem ihrer Vielfachen entspricht, wobei die (n+n/2) Festbettreaktoren jeweils mindestens ein Festbett mit einem Katalysator (Cb) umfassen, wobei die (n+n/2) Festbettreaktoren derart parallel und abgestuft arbeiten, dass der Reaktionsschritt in jedem der Reaktoren mit einer Zeitverschiebung beginnt, die einem Viertel der Dauer des Katalysezyklus des Katalysators (Cb) entspricht, wobei der Reaktions-/Regenerationsabschnitt drei Regenerationsdurchläufe umfasst, und derart, dass zu jedem Zeitpunkt:
b1) der Reaktionsschritt in n der Festbettreaktoren, wobei n eine Ganzzahl ist, die 4 oder einem ihrer Vielfachen entspricht, in die ein Teil des Ethanol/Acetaldehyd-Austrags aus Schritt a) geleitet wird, bei einer Temperatur zwischen 300 und 400°C, bei einem Druck zwischen 0,1 und 1,0 MPa und während einer Dauer betrieben wird, die der Dauer des Katalysezyklus des Katalysators (b) entspricht, zum Herstellen eines Reaktionsaustrags, und
b2) der Regenerationsschritt in n/2 der Festbettreaktoren während einer Gesamtdauer betrieben wird, die der Hälfte der Dauer des Katalysezyklus des Katalysators (Cb) entspricht, und die vier folgenden aufeinanderfolgenden Phasen umfasst:
i. eine Strippingphase, die bei einer Temperatur zwischen 300 und 400°C in einem Inertgasstrom betrieben wird, wobei mit der Phase i) am Ende von Reaktionsschritt b1) begonnen wird; dann
ii. eine erste Verbrennungsphase, die am Ende von Phase i) in einem Gasstrom, der das Inertgas und Sauerstoff mit einem Gehalt kleiner gleich 1°Vol.-% bezogen auf das Gesamtvolumen des Gasstroms umfasst, bei einer Temperatur zwischen 300 und 450°C betrieben wird; dann
iii. eine zweite Verbrennungsphase, die am Ende der ersten Verbrennungsphase ii) in einem Gasstrom, der das Inertgas und Sauerstoff mit einem Gehalt größer gleich 2°Vol.-% bezogen auf das Gesamtvolumen des Gasstroms umfasst, bei einer Temperatur zwischen 390 und 550°C betrieben wird; dann
iv. eine abschließende Strippingphase, die bei einer Temperatur zwischen 550°C und 300°C in einem Strom des Inertgases betrieben wird;
wobei die drei Regenerationsdurchläufe des Reaktions-/Regenerationsabschnitts einen Durchlauf zur Regeneration des Inertgases, einen Durchlauf zur Regeneration des Gasstroms der ersten Verbrennungsphase ii) und einen Durchlauf zur Regeneration des Gasstroms der zweiten Verbrennungsphase iii) umfassen.

2. Verfahren nach Anspruch 1, wobei der Reaktionsabschnitt von Schritt a) bei einer Temperatur zwischen 200 und 500°C, vorzugsweise zwischen 250°C und 300°C und bei einem Druck zwischen 0,1 und 1,0 MPa, vorzugsweise zwischen 0,1 und 0,5 MPa, bevorzugter zwischen 0,1 und 0,3 MPa betrieben wird.

3. Verfahren nach einem der Ansprüche 1 bis 2, wobei in die Festbettreaktoren, die in dem Reaktionsschritt b1) eingesetzt werden, ferner eine Zusatzmenge Ethanol und/oder eine Zusatzmenge Acetaldehyd geleitet wird bzw. werden, wobei der Durchfluss der eingeleiteten Stoffe derart ist, dass das Molverhältnis zwischen der Gesamtmolmenge Ethanol bezogen auf die Gesamtmolmenge Acetaldehyd am Einlass der Festbettreaktoren von Reaktionsschritt b1) zwischen 1 und 5 liegt.

4. Verfahren nach einem der Ansprüche 1 bis 3, wobei die Ganzzahl n 4 entspricht und der Reaktions-/Regenerationsabschnitt von Schritt b) 6 Festbettreaktoren umfasst.

5. Verfahren nach einem der Ansprüche 1 bis 4, wobei der Reaktionsschritt b1) bei einer Temperatur zwischen 300 und 360°C betrieben wird.

6. Verfahren nach einem der Ansprüche 1 bis 5, wobei der Reaktionsschritt b1) bei einem Druck zwischen 0,2 und 0,4 MPa betrieben wird.

7. Verfahren nach einem der Ansprüche 1 bis 6, wobei die Dauer des Katalysezyklus des Katalysators (Cb) von Schritt b) zur Umwandlung in Butadien länger als oder gleich 1 Tag, vorzugsweise länger als oder gleich 6 Tage, und kürzer als oder gleich 20 Tage, besonders bevorzugt kürzer als oder gleich 15 Tage beträgt.

8. Verfahren nach einem der Ansprüche 1 bis 7, wobei das Inertgas des Regenerationsschritt b2) Stickstoff, Kohlendioxid (CO₂) oder ihre Mischungen, sehr bevorzugt Stickstoff ist.

9. Verfahren nach einem der Ansprüche 1 bis 8, wobei die Strippingphase i) bei einer Temperatur zwischen 330 und 370°C betrieben wird.

10. Verfahren nach einem der Ansprüche 1 bis 9, wobei der Inertgasdurchfluss in der Strippingphase i) zwischen 0,5 und 1,5 Nm³/h/kg Katalysator, vorzugsweise 1 Nm³/h/kg Katalysator beträgt.

11. Verfahren nach einem der Ansprüche 1 bis 10, wobei die erste Verbrennungsphase ii) in einem Gasstrom betrieben wird, der bezogen auf das Gesamtvolumen des Gasstroms einen Sauerstoffgehalt zwischen 0,1 und 1°Vol.-%, vorzugsweise zwischen 0,3 und 0,7°Vol.-% aufweist.

12. Verfahren nach einem der Ansprüche 1 bis 11, wobei die erste Verbrennungsphase ii) bei einer Temperatur zwischen 330 und 430°C, insbesondere bei einer konstanten Temperatur zwischen 330 und 370°C, gefolgt von einem Temperaturanstieg von 10 bis 30°C/h, dann einer Phase mit einer konstanten Temperatur zwischen 390 und 430°C betrieben wird.

13. Verfahren nach einem der Ansprüche1 bis 12, wobei die erste Verbrennungsphase ii) bei einem Durchfluss des Gasstroms zwischen 1,7 und 2,5 Nm³/h/kg Katalysator, vorzugsweise zwischen 1,9 und 2,3 Nm³/h/kg Katalysator betrieben wird.

14. Verfahren nach einem der Ansprüche 1 bis 13, wobei die zweite Verbrennungsphase iii) in einem Gasstrom betrieben wird, der bezogen auf das Gesamtvolumen des Gasstroms einen Sauerstoffgehalt zwischen 2 und 20°Vol.-%, vorzugsweise zwischen 2 und 10 Vol.-%, bevorzugt zwischen 4 und 8°Vol.-% aufweist.

15. Verfahren nach einem der Ansprüche 1 bis 14, wobei die zweite Verbrennungsphase iii) bei einer konstanten Temperatur zwischen 390 und 430°C, gefolgt von einem Temperaturanstieg von 10 bis 30°C/h, dann einer Phase mit einer konstanten Temperatur zwischen 460 und 510°C betrieben wird.

16. Verfahren nach einem der Ansprüche 1 bis 15, wobei die zweite Verbrennungsphase iii) bei einem Durchfluss des Gasstroms zwischen 1,2 und 1,8 Nm³/h/kg Katalysator, vorzugsweise zwischen 1,35 und 1,50 Nm³/h/kg Katalysator betrieben wird.

17. Verfahren nach einem der Ansprüche 1 bis 16, wobei die abschließende Strippingphase iv) mit einem Temperaturrückgang von 50 bis 150°C/h, gefolgt von einer Phase mit einer konstanten Temperatur zwischen 300 und 400°C, vorzugsweise zwischen 330 und 370 C betrieben wird.

18. Verfahren nach einem der Ansprüche 1 bis 17, wobei die abschließende Strippingphase iv) mit einem Strom des Inertgases bei einem Durchfluss zwischen 0,5 und 1,5 Nm³/h/kg Katalysator, vorzugsweise gleich 1 Nm³/h/kg Katalysator betrieben wird.

## Claims

1. Process for producing butadiene from ethanol, comprising at least the following steps:
a) a step of converting ethanol into acetaldehyde, to produce an ethanol/acetaldehyde effluent, comprising at least one reaction section (A) fed with a stream comprising ethanol and operated in the presence of a catalyst (Ca);
b) a butadiene conversion step comprising at least one reaction-regenerative section in which are simultaneously performed a reaction step and a regeneration step in (n+n/2) fixed-bed reactors, n being an integer equal to 4 or a multiple thereof, said (n+n/2) fixed-bed reactors each comprising at least one fixed bed of a catalyst (Cb), said (n+n/2) fixed-bed reactors functioning in parallel and in sequence so that said reaction step starts in each of said reactors with a time shift equal to a quarter of the catalytic cycle time of said catalyst (Cb), said reaction-regenerative section comprising three regeneration loops, and so that, at each instant:
b1) said reaction step is operated in n of said fixed-bed reactors, n being an integer equal to 4 or a multiple thereof, fed at least with a fraction of said ethanol/acetaldehyde effluent obtained from step a), at a temperature of between 300 and 400°C, at a pressure of between 0.1 and 1.0 MPa, and for a time equal to the catalytic cycle time of said catalyst (b), to produce a reaction effluent, and
b2) said regeneration step is operated in n/2 of said fixed-bed reactors for a total time equal to half of the catalytic cycle time of said catalyst (Cb), and comprises the following four successive phases:
i. a stripping phase operated at a temperature of between 300 and 400°C, under a stream of inert gas, said phase i) starting on conclusion of the reaction step b1); and then
ii. a first combustion phase operated on conclusion of phase i) under a gas stream comprising said inert gas and oxygen in a content of less than or equal to 1 vol% relative to the total volume of said gas stream, at a temperature of between 300 and 450°C; and then
iii. a second combustion phase operated on conclusion of the first combustion phase ii) under a gas stream comprising said inert gas and oxygen in a content of greater than or equal to 2 vol% relative to the total volume of said gas stream, at a temperature of between 390 and 550°C; and then
iv. a final stripping phase operated at a temperature of between 550°C and 300°C, under a stream of said inert gas;
said three regeneration loops of said reaction-regenerative section comprising a regeneration loop for the inert gas, a regeneration loop for the gas stream of the first combustion phase ii) and a regeneration loop for the gas stream of the second combustion phase iii).

2. Process according to Claim 1, in which the reaction section of step a) is operated at a temperature of between 200 and 500°C, preferentially between 250°C and 300°C, and at a pressure of between 0.1 and 1.0 MPa, preferentially between 0.1 and 0.5 MPa, more preferably between 0.1 and 0.3 MPa.

3. Process according to either of Claims 1 and 2, in which said fixed-bed reactors used in said reaction step b1) are also fed with a supply of ethanol and/or a supply of acetaldehyde, the feed flow rates being such that the mole ratio between the total molar amount of ethanol relative to the total molar amount of acetaldehyde entering said fixed-bed reactors of said reaction step b1) is between 1 and 5.

4. Process according to one of Claims 1 to 3, in which the integer n is equal to 4 and said reaction-regenerative section of step b) comprises six fixed-bed reactors.

5. Process according to one of Claims 1 to 4, in which said reaction step b1) is operated at a temperature of between 300 and 360°C.

6. Process according to one of Claims 1 to 5, in which said reaction step b1) is operated at a pressure of between 0.2 and 0.4 MPa.

7. Process according to one of Claims 1 to 6, in which the catalytic cycle time of said catalyst (Cb) for the butadiene conversion step b) is greater than or equal to 1 day, preferably greater than or equal to 6 days, and less than or equal to 20 days, more particularly preferably less than or equal to 15 days.

8. Process according to one of Claims 1 to 7, in which the inert gas of the regeneration step b2) is nitrogen, carbon dioxide (CO₂) or mixtures thereof, very preferably nitrogen.

9. Process according to one of Claims 1 to 8, in which said stripping phase i) is operated at a temperature of between 330 and 370°C.

10. Process according to one of Claims 1 to 9, in which the flow rate of inert gas of said stripping phase i) is between 0.5 and 1.5 Nm³/h/kg of catalyst, preferably equal to 1 Nm³/h/kg of catalyst.

11. Process according to one of Claims 1 to 10, in which said first combustion phase ii) is operated under a gas stream comprising an oxygen content of between 0.1 and 1 vol%, preferably between 0.3 and 0.7 vol%, relative to the total volume of said gas stream.

12. Process according to one of Claims 1 to 11, in which said first combustion phase ii) is operated at a temperature of between 330 and 430°C, in particular at a constant temperature of between 330 and 370°C followed by a temperature increase ramp of 10 to 30°C/h and then a phase at a constant temperature of between 390 and 430°C.

13. Process according to one of Claims 1 to 12, in which said first combustion phase ii) is operated at a flow rate of gas stream of between 1.7 and 2.5 Nm³/h/kg of catalyst, preferably between 1.9 and 2.3 Nm³/h/kg of catalyst.

14. Process according to one of Claims 1 to 13, in which said second combustion phase iii) is operated under a gas stream comprising an oxygen content of between 2 and 20 vol%, preferably between 2 and 10 vol%, preferentially between 4 and 8 vol%, relative to the total volume of said gas stream.

15. Process according to one of Claims 1 to 14, in which said second combustion phase iii) is operated at a constant temperature of between 390 and 430°C followed by a temperature increase ramp of 10 to 30°C/h and then a phase at a constant temperature of between 460 and 510°C.

16. Process according to one of Claims 1 to 15, in which said second combustion phase iii) is operated at a flow rate of gas stream of between 1.2 and 1.8 Nm³/h/kg of catalyst, preferably between 1.35 and 1.50 Nm³/h/kg of catalyst.

17. Process according to one of Claims 1 to 16, in which said final stripping phase iv) is operated on a temperature decrease ramp of 50 to 150°C/h followed by a phase at a constant temperature of between 300 and 400°C, preferably between 330 and 370°C.

18. Process according to one of Claims 1 to 17, in which said final stripping phase iv) is operated under a stream of said inert gas, at a flow rate of between 0.5 and 1.5 Nm³/h/kg of catalyst, preferably equal to 1 Nm³/h/kg of catalyst.
